# EUROPÄISCHE PATENTSCHRIFT

(11) **EP 0 930 909 B1**
(45) Veröffentlichungstag und Bekanntmachung des Hinweises auf die Patenterteilung: **21.05.2003**
(21) Anmeldenummer: 97941996.7
(22) Anmeldetag: 08.09.1997
(51) Int. Cl.: A61M 16/04

(54) **TRACHEALTUBUS**
TRACHEAL TUBE
TUBE ENDOTRACHEAL

(30) Priorität: 10.09.1996 DE 19636654; 23.09.1996 DE 19638935
(43) Veröffentlichungstag der Anmeldung: 28.07.1999
(73) Patentinhaber: Dr. Fred Göbel Patentverwaltung GmbH, 69115 Heidelberg (DE)
(72) Erfinder: Göbel, Fred G., 93047 Regensburg (DE)
(74) Vertreter: Grünecker, Kinkeldey, Stockmair & Schwanhäusser Anwaltssozietät
(86) Internationale Anmeldenummer: EP9704891
(87) Internationale Veröffentlichungsnummer: WO98010819

(56) Entgegenhaltungen:
- FR-A- 1 561 588
- GB-A- 1 060 629
- GB-A- 1 133 728
- US-A- 4 423 725
- US-A- 5 505 698

## Beschreibung

Die Erfindung bezieht sich auf einen Trachealtubus, der die Trachea flüssigkeitsdicht verschließt, mit einer die Trachea unterhalb der Glottis blockierenden Fixier-Cuffmanschette, durch die eine Beatmungskanüle durchgeführt ist, wobei cranial, oberhalb der Cuffmanschette eine durch Zufuhr von Fluid vergrößerbare Tamponierblase aus flexiblem Material angeordnet ist, der in Gestalt in gefülltem Zustand verschieden von der Gestalt der Cuffmanschette ist.

Bei einem in der US 4,235,239 beschriebenen Trachealtubus dieser Gattung ist die Cuffmanschette in aufgeblasenem Zustand etwa kugelförmig gestaltet und ist eine an der Glottis anliegende Verankerung. Die Cuffmanschette ist so ausgebildet, daß der subglottische Raum frei bleibt. Beabstandet oberhalb der Cuffmanschette ist eine zweite Cuffmanschette vorgesehen, die aufgeblasen etwa scheibenförmige Gestalt hat und unter Druck flüssigkeitsdicht oberhalb der Glottis nahe dem Rachenraum anliegt. Bei diesem Trachealtubus wird die Glottis zwischen den beiden Cuffmanschetten zusammengequetscht, so daß sich bei längerer intubation gefährliche Quetschungen und Nekrosen (Gewebeschäden durch Absterben von Gewebeteilen) ergeben können. Femer besteht dabei die Gefahr der Luxation (des Herausgleitens) des Tubus.

In der US 5,033,466 ist ein Trachealtubus bekannt, bei dem eine Cuffmanschette stark aufgeblasen wird und unter Druck an der Luftröhre anliegt, um einen flüssigkeitsdichten Abschluß zu schaffen. Beabstandet oberhalb von der Cuffmanschette ist eine mit Schaumstoff gefüllte zweite Manschette im engsten Querschnitt der Glottis vorgesehen, damit die härtere Beatmungskanüle an der Glottis nicht anschlägt und sie beschädigt.

Bei der Verwendung einer einzelnen Cuffmanschette machen es die niedrigen Perfusionsdrücke im epithelialen Gefäßbett der subglottischen Schleimhaut (Mucosa) bei der endotrachealen Intubation erforderlich, daß die Cuffmanschette des Tubus über den Ringknorpel des Kehlkopfes hinweg bis mindestens in den Bereich der oberen Trachea vorgeschoben wird. Es ergibt sich somit, intubationsbedingt, ein vom oberen Cuffmanschettenrand bis zu den Stimmlippen reichender sogenannter subglottischer Raum. Während der Intubation kann bakteriell hochkontaminiertes Sekret des Rachens ungehindert in den subglottischen Raum einfließen und dort, therapeutsch nur schwer zugänglich, inkubieren.

Das subglottische Sekret stellt für den beatmeten Patienten eine Gefährdung in zweierlei Hinsicht dar.

Zum einen gelangt im Verlaufe einer Beatmungstherapie aufgrund der unzureichenden Abdichtung der momentan eingesetzten Cuffmanschetten fortwährend Sekret über die Cuffbarriere hinweg in das distale tracheobronchiale System, und ist so für die Entstehung des überwiegenden Teils aller beatmungsassoziierter Pneumonien (Lungenentzündungen) verantwortlich.

Zum andern führt die Stase (Stauung) des subglottischen Sekrets bereits nach wenigen Tagen zu entzündlichen Veränderungen der dortigen Schleimhaut. Der chronische Entzündungsprozeß kann zu erheblichen Folgekomplikationen, wie z.B. der Entwicklung subglottischer Stenosen führen.

In der US 4,091,816 ist ein Trachealtubus mit zwei fluidverbundenen Cuffmanschetten offenbart. Die untere Cuffmanschette ist kugelförmig, unelastisch und liegt als Verankerung von unten an der Glottis an. Die Manschetten sind über eine Nut voneinander beabstandet, damit die Glottis zwischen den beiden Cuffs eingreifen kann. Die obere Cuffmanschette liegt oberhalb der Glottis und kann sich in den Rachenraum hin erweitern. Bei der Verwendung dieses Trachealtubus besteht die Gefahr, daß die Glottis durch Quetschung verletzt wird. Die Ausdehnung des oberen Cuffs im Rachen kann zu autonomen Irritationen, wie z.B. zu vagalen Reflexen, führen und bereitet daher während der langwierigen Phase der Entwöhnung von einem Respirator Schwierigkeiten.

In der US 4,449,523 wird ein Trachealtubus vorgeschlagen, bei dem eine erste Cuffmanschette flüssigkeitsdicht in der Luftröhre aufgeblasen wird. Beabstandet von der Cuffmanschette befindet sich eine zweite Cuffmanschette zur Abdichtung des Lochs im Halse zum Durchführen der Beatmungskanüle. Zwischen den Cuffmanschetten sind Luftlöcher inder Kanüle vorgesehen. Dies birgt die Gefahr, daß hochkontaminiertes Sekret durch die Löcher angeatmet wird.

In der EP 0 277 797 A2 ist ein Operationstubus für Laseroperationen beschrieben. An einer Beatmungskanüle sind zwei gleich ausgebildete Cuffmanschetten angeordnet. Beide Manschetten werden für die Dauer des Eingriffs geblockt, um unter Druck flüssigkeitsdicht an der Luftröhre anzuliegen. Die obere Manschette ist als Puffer gegen mögliche Verletzungen durch ein Laserinstrument gedacht. Dieser Operationstubus ist nur für kurzzeitige Intubation während Operationen geeignet. Bei Langzeitintubation bestünde die Gefahr, daß sich aufgrund der bewußt minimalisierten Oberfläche des Lasertubus und der damit verbundenen ungünstigen Druckentwicklung auf das Epithel rasch Läsionen der Trachealschleimhaut entwickein.

In der DE 295 11 468 U1 und der G 87 115 92.1 sind Trachealtuben mit zwei beabstandet zueinander angeordneten und gleich ausgebildeten Cuffmanschetten beschrieben.

Der Erfindung liegt die Aufgabe zugrunde, einen Trachealtubus der eingangs genannten Gattung dahingehend zu verbessern, daß ein Patient damit möglichst schonend über lange Zeit intubiert werden kann, wobei das Infektionsrisiko gering sein soll.

Diese Aufgabe wird erfindungsgemäß mit einem Trachealtubus mit den Merkmalen des Anspruchs 1 gelöst.

Durch die sich unmittelbar an die distale Cuffmanschette anschließende Tamponierblase wird das subglottische Erregerreservoir praktisch eliminiert bzw. auf einen schmalen Saum zwischen der Trachealschleimhaut und dem Verdrängungskörper, das heißt der Tamponierblase reduziert. Durch die unmittelbare Anlage der Tamponierblase an der Cuffmanschette ergibt sich auch in diesem Bereich ein im wesentlichen kontinuierlicher Übergang, in dem das Erregerreservoir nur auf den schmalen Saum reduziert ist. Im Prinzip ergibt sich kein Erregerreservoir, sondern nur ein dünner Sekretfilm.

Rachensekret wird in den supraglottischen Bereich zurückgedrängt bzw. am Vordringen in den subglottischen Bereich weitgehend gehindert. Der Infektionsweg für Keime des Rachensekrets wird durch den Verdrängungskörper maximal verlängert und reicht bei entfalteter Tamponierblase von der Glottis bis zum unteren Rand der Cuffmanschette. Erreger benötigen damit wesentlich länger zum Passieren des Infektionswegs und können im Bereich des schmalen Saums zwischen der Vorrichtung und der Schleimhaut durch epitheleigene Abwehrmechanismen neutralisiert und so am Vordringen in die distalen Luftwege gehindert werden. Die erfindungsgemäße Lösung ermöglicht also erstmals, die körpereigenen Abwehrfunktionen zum Vermeiden von Entzündungen zu nutzen.

Da bei entfalteter Tamponierblase kein infektiologisch relevantes subglottisches Sekretvolumen bestehen bleibt, kann so das Risiko chronisch entzündlich bedingter subglottischer Stenosen ebenfalls ausgeschlossen werden.

Da die Tamponierblase lediglich zum Ausfüllen des subglottischen Raums dient, kann sie zur Erfüllung ihrer Funktion mit einem epithelschonenden minimalen Druck beaufschlagt werden. Druckbedingte Nekrosen im Bereich der subglottischen Schleimhaut können so vermieden werden.

Vorteilhafte Merkmale und Ausführungsformen der Erfindung sind in den abhängigen Ansprüchen genannt.

Die Erfindung wird nachstehend anhand der Figuren an Ausführungsbeispielen näher erläutert. Es zeigen:
- Fig. 1: ein Frontalschnitt durch den Kehlkopf, mit den angrenzenden anatomischen Strukturen, sowie darin eingezeichnet, ein nasaler bzw. oraler Endotrachealtubus mir regulärer Cuffmanschette und sich darüber anschließendem Tamponierballon,
- Fig. 2: den Aufbau der Vorrichtung mit Trachealtubus, Cuff, Tamponierballon und extrakorporalem Reservoir in einer bevorzugten Ausführungsform der Erfindung,
- Fig. 2a: einen Druckausgleichsballon in nicht gefülltem Zustand für einen erfindungsgemäßen Trachealtubus,
- Fig. 2b: den Druckausgleichsballon von Figur 2b in vergrößertem, gefüllten Zustand,
- Fig. 3: ein Detail der Erfindung,
- Fig. 3a: einen vergrößerten Ausschnitt von Cuffmanschette und damit verklebter Tamponierblase gemäß einer besonderen Ausführungsform,
- Fig. 4: eine Variation des Details von Figur 3,
- Fig. 5: ein weiteres Detail der Erfindung.

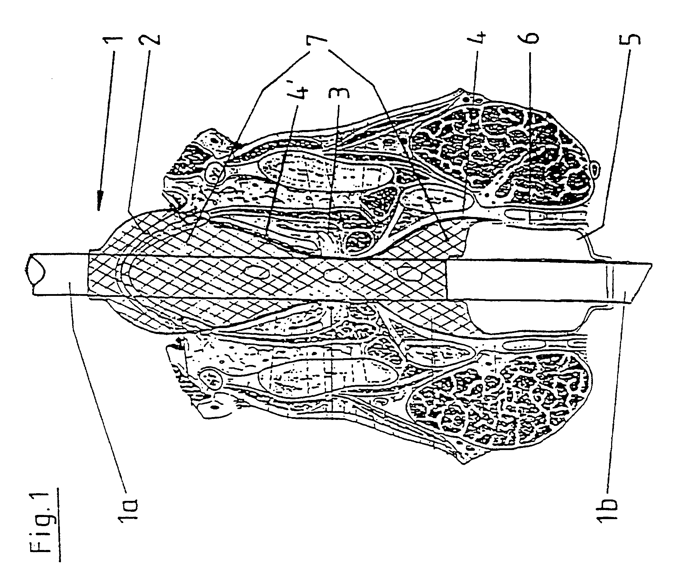

In der Figur 1 sind ein korrekt plazierter endotrachealer Trachealtubus 1 und die topographischen Beziehungen zu den relevanten angrenzenden anatomischen Strukturen (Kehlkopf und obere Luftröhre) dargestellt. Der Trachealtubus 1 passiert von cranial 1a nach caudal 1b den Kehldeckel 2 (Epiglottis), den Bereich der Stimmlippen 3 (Glottis), und den sich an die Stimmlippen anschließenden subglottischen Raum 4, der nach caudal hin vom oberen Rand der Cuffmanschette 5 begrenzt ist. Die Fixierung des Trachealtubus 1 in der Luftröhre erfolgt distal des Ringknorpels, mindestens im Bereich der ersten oberen Trachealspangen 6 durch eine reguläre luftgeblockte Cuffmanschette 5. Um eine accidentelle Extubation des Patienten zu vermeiden, wird vom klinischen Anwender oft auch eine Blockade des Tubus im mittleren trachealen Drittel bevorzugt.

Mit 7 ist ein mit Wasser (oder einem anderen geeigneten Medium) gefüllter Tamponierballon 7 bezeichnet (schraffiert dargestellt), der sich an den oberen Rand der Cuffmanschette nach cranial hin anschließt, und somit den subglottischen Raum 4 ausfüllt. Die Tamponierblase 7 liegt im Berührungsbereich mit der Cuffmanschette in Richtung quer zur Beatmungskanüle unmittelbar eng an der Cuffmanschette an. Der Tamponierballon 7 erstreckt sich nach cranial wahlweise bis in den Bereich der Stimmlippen 3 (Glottis), bis in den supraglottischen Bereich 2 (Vestibulum epiglotticum), oder bis in den unteren Rachen (Hypopharynx).

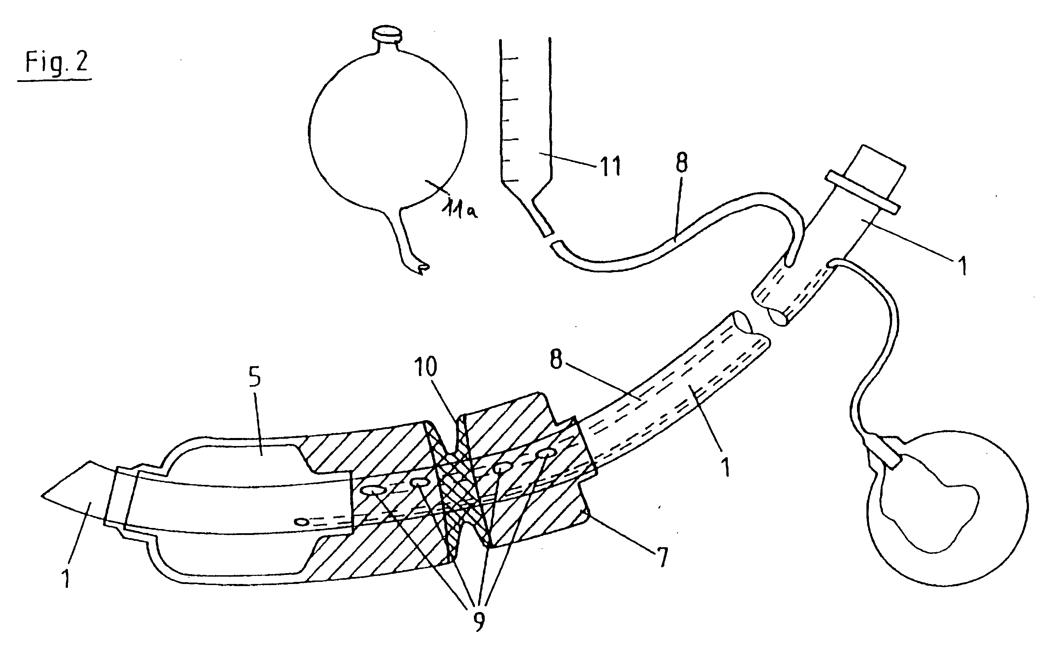

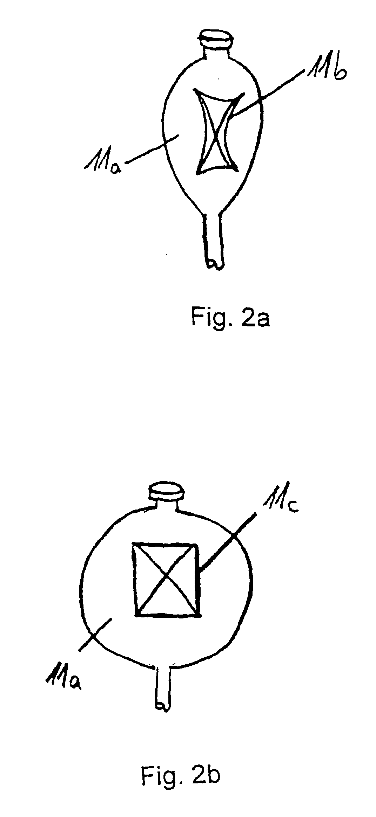

In Figur 2 ist das funktionelle der Konzept der Erfindung bei einer bevorzugten Ausführungsform dfes gesamten Trachealtubus näher dargestellt. Über einen in die Wand eines, mit einer konventionellen High-Volume/Low-Pressure Cuffmanschette 5 versehenen Trachealtubus 1, eingearbeiteten, möglichst großlumigen dritten Kanal 8, kann der Tamponierballon 7 über mehrere Austrittsöffnungen 9 mit Wasser (oder einem anderen geeigneten Medium, wie zum Beispiel Gas) geflutet werden.

Der Tamponierballon 7 besteht aus einem folienartigen, äußerst dehnbaren Material, das sich ohne Faltenwurf eng an die Wandungen des Kehlkopfes anschmiegt. Um eine korrekte Lage des Trachealtubus 1 und seines Tamponierballons 7 zu gewährleisten, ist der Tamponierballon 7 in dem Bereich durch eine farbige Markierung 10 gekennzeichnet, die der späteren Lage zwischen den Stimmlippen 3 entspricht.

Über den Kanal 8 ist die Wasserfüllung der Tamponierblase mit einer außerhalb des Patienten angebrachten, skalierten Wassersäule 11 großlumig verbunden. Nach dem Prinzip kommunizierender Röhren sorgt dieses Reservoir für eine fortwährende, formerhaltende Füllung des Tamponierballons 7, ohne dabei die Kehlkopfstrukturen selbst unter Spannung zu nehmen.

In einer anderen in Figur 2, 2a und 2b gezeigten Ausführungsform kann die offene skalierte Wassersäule 11 durch einen flexiblen Druckausgleichsballon 11 a ersetzt werden, der mittels seiner Wandspannung einen lediglich formerhaltenden Druck innerhalb der subglottischen Tamponierblase aufrechterhält. Der Ballon 11a ist z.B. derart gestaltet, daß sich ein im nicht gefüllten Zustand auf seine Wandung aufgedrucktes Muster 11b bei korrekter Füllung zu einer symmetrischen geometrischen Figur 11c aufdehnt und so den optimalen Fülldruck anzeigt. Anstatt des aufgedruckten Musters 11b kann auch eine präformierte Formgestalt vorgesehen sein, die im ungefüllten Zustand verformt ist (Fig. 2a) und bei korrekter Füllung zu einer symmetrisch geometrischen Figur ausgeformt ist und so den optimalen Fülldruck anzeigt (Fig. 2b).

Tamponierblase und Ausgleichsballon können mit flüssigen oder gasförmigen Medien befüllt werden.

Die Tamponierblase 7 ist mit dem Trachealtubus 1 beispielsweise durch Verkleben, Schrumpfen und Verschweißen, Bandagieren oder dergleichen verbunden.

Das Material der Tamponierblase 7 besteht vorzugsweise aus einer körperverträglichen Weichfolie mit latexhautähnlichen Eigenschaften. Sie kann sich den anathomischen Strukturen des subglottischen Raumes.4 bei minimaler Wandspannung im wesentlichen ohne Faltenwurf anschmiegen. Sie kann in ihrer Form dem subglottischen Raum 4 entsprechend zusätzlich präformiert sein.

Gemäß einer weiteren Ausführungsform kann die Tamponierblase derart gestaltet sein, daß der Durchmesser der nicht gefüllten Tamponierblase geringfügig größer als der des subglottischen Raumes ist. Das Aufdehnen der Tamponierblase erübrigt sich somit, was die Tamponade des subglottischen Raumes bei noch geringeren Fülldrücken epithelschonend gestattet. Bei Verwendung entsprechend dünner Wandmaterialen begünstigt der entstehende Faltenwurf das Absteigen von Sekreten nicht.

Wahlweise kann das Material der Tamponierblase bei Faltenwurf leicht an sich selbst haften, so daß es sich bei Faltenwurf automatisch dicht an sich selbst anschmiegt. Dieses leichte Anhaften ist beispielsweise mit dem Anhaften von dünner Haushaltsfolie aus Kunststoff vergleichbar.

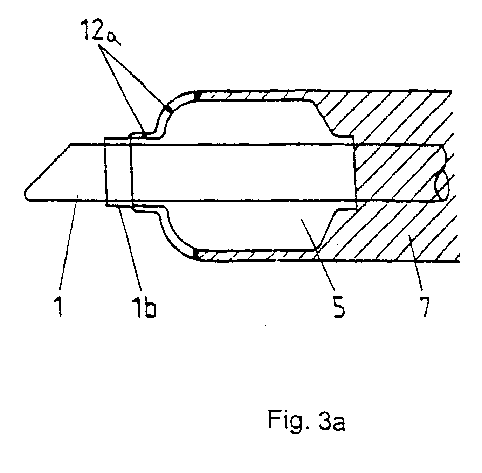

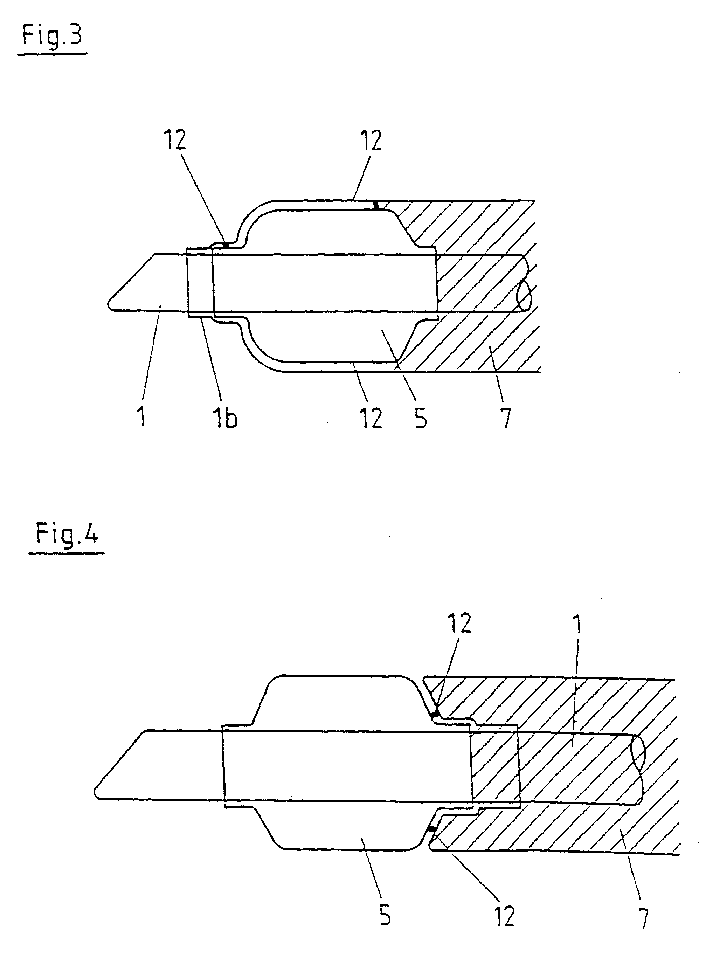

Wie in Figur 3 dargestellt, ist die Tamponierblase 7 in einer Ausführungsform der Erfindung über die Cuffmanschette 5 vollständig hinweggezogen und am unteren, caudalen Ende des Trachealtubus 1 b mit diesem verbunden bzw. abgeschlossen. Um Verlagerungen des Ballons 7 nach caudal, sogenannte Hemiationen auszuschließen, kann die Cuffmanschette 5 im Bereich der gemeinsamen Auflagefläche 12 mit der Tamponierblase 7 verklebt sein.

Wahlweise kann die Cuffmanschette 5 nur im Bereich der gemeinsamen distalen Auflagefläche 12a verklebt sein. Damit wird eine bessere separate Inflation von Cuffmanschette 5 und Tamponierblase ermöglicht. Dementsprechend kann die Verklebung nur in der in aufgeblasenem Zustand quer zu dem Trachealtubus 1 verlaufenden gemeinsamen Auflagefläche 12a verklebt sein, wie in Figur 3a gezeigt. Die parallel zu dem Trachealtubus 1 verlaufende gemeinsame Auflagefläche ist nicht verklebt.

Bei einer anderen, in Figur 4 gezeigten, Ausführungsform der Erfindung, schließt sich die Fixierung der Tamponierblase 7 nach cranial unmittelbar an den oberen Rand der Cuffmanschette 5 an. Im Bereich ihrer gemeinsamen Auflagefläche sind die Cuffmanschette 5 und die Tamponierblase 7 bei 12 verklebt.

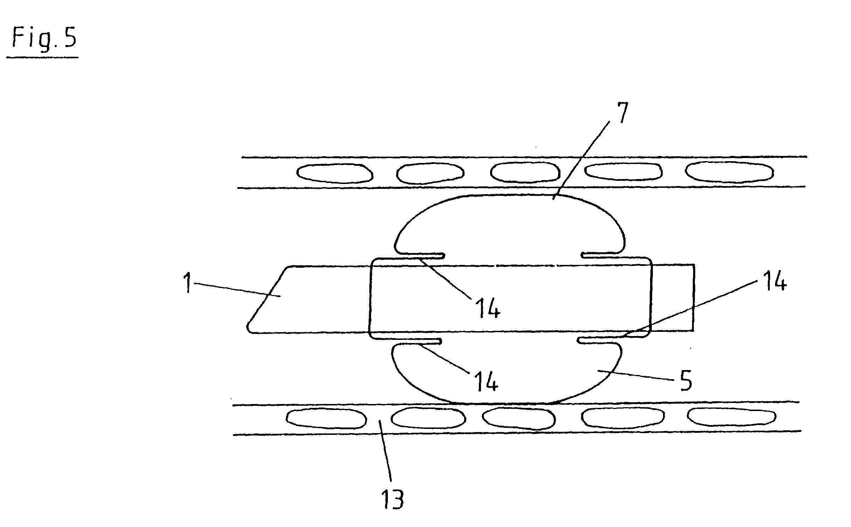

Um unerwünschte Scherbewegungen des Cuffs 5 gegen die Trachealwand 13, wie sie sich zum Beispiel beim Schluckakt ereignen, weitestgehendst zu minimieren, ist der Trachealtubus 1 bevorzugt innerhalb der Cuffmanschette 5 zudem (bei allen Varianten) in einem gewissen Umfang durch eine Umschlagfalte 14 im Bereich der Manschettenfixierung am Trachealtubus, beweglich gelagert sein (Hierzu Figur 5).

Das Prinzip der oben aufgeführten sub- oder supraglottische Tamponade kann auch ohne begleitenden Beatmungstubus, sondern lediglich über eine alleinstehende Schlauchverbindung mit einem extrakorporalen Reservoir verwirklicht werden. Ein derartiger Schutz des Kehlkopfes und der Trachea gegen abfließende Sekrete, macht die Erfindung auch für den tracheotomierten und über eine Trachealkanüle beatmeten Patienten nutzbar.

Den Ausführungsformen mit der Wassersäule ist gemeinsam, daß der Wasserdruck in der Tamponierblase 7 bzw. 16 allein durch die Wassersäule des bis in das Reservoir 11 anstehenden Wassers erreicht wird, wobei sich das Reservoir 11 auf einem Niveau etwas oberhalb der Tamponierblase 7 bzw. 16 befindet. Hierdurch wird ein geringfügig über dem Atmosphärendruck liegender, sehr konstanter Innendruck für die Tamponierblase 7 bzw. 16 erreicht.

### Bezugszeichenliste

- 1: Trachealtubus
- 2: Kehldeckel (Epiglottis)
- 3: Stimmlippen (Glottis)
- 4: subglottischer Raum
- 4': supraglottischer Raum
- 5: Cuffmanschette
- 6: Trachealspangen
- 7, 16: Tamponierblase
- 8: Kanal
- 9: Austrittsöffnung
- 10: Markierung
- 11: Reservoir
- 11a: Ballon
- 11b: Muster
- 11c: Figur
- 12: Auflagefläche
- 12a: Auflagefläche
- 13: Trachealwand
- 14: Umschlagfalte

## Patentansprüche

1. Trachealtubus, der die Trachea flüssigkeitsdicht verschließt, mit einer die Trachea unterhalb der Glottis (3) blockierenden Fixier-Cuffmanschette (5), durch die eine Beatmungskanüle (1) durchgeführt ist, wobei cranial, oberhalb der Cuffmanschette (5) eine durch Zufuhr von Fluid vergrößerbare Tamponierbtase (7) aus flexiblem Material angeordnet ist, die in Gestalt in gefülltem Zustand verschieden von der Gestalt der Cuffmanschette (5) ist,
**dadurch gekennzeichnet,**
**daß** die Tamponierblase (7) in gefülltem Zustand unmittelbar an der Cuffmanschette (5) anliegt und aus folienartigem Material besteht und in ihrer Größe in gefülltem Zustand zum Ausfüllen des subglottischen Raumes ausgebildet ist.

2. Trachealtubus nach Anspruch 1,
**dadurch gekennzeichnet,**
**daß** die Tamponierblase (7) derart ausgebildet ist, daß sie sowohl den subglottischen als auch den supraglottischen Raum ausfüllt.

3. Trachealtubus nach Anspruch 1 oder 2,
**dadurch gekennzeichnet,**
**daß** die Tamponierblase (7) preformiert und der inneren Morphologie des Kehlkopfes und der Glottis angepaßt ist.

4. Trachealtubus nach Anspruch 3,
**dadurch gekennzeichnet,**
**daß** die Tamponierblase (7) ausgehend von der Cuff-Manschette (5) einen sich verengenden Querschnitt und daran anschließend vorzugsweise innen sich wieder erweiternden Querschnitt aufweist.

5. Trachealtubus nach einem der vorhergehenden Ansprüchen,
**dadurch gekennzeichnet,**
**daß** die Tamponierblase an einem ein Plazieren der Blase ermöglichenden schlauchartigen Plazierelement vorgesehen ist.

6. Trachealtubus nach einem der vorhergehenden Ansprüche,
**dadurch gekennzeichnet,**
**daß** die Tamponierblase (7) mit ihrer Wandung die Cuffmanschette (5) umschließt.

7. Trachealtubus nach einem der vorhergehende Ansprüche,
**dadurch gekennzeichnet,**
**daß** die Wandung der Tamponierblase (7) mit der Wandung der Cuffmanschette, zumindest in einem Teilbereich ihrer gemeinsamen Anlagefläche, vorzugsweise durch Verkleben, verbunden ist.

8. Trachealtubus nach Anspruch 7,
**dadurch gekennzeichnet,**
**daß** die Tamponierblase (7) und die Cuffmanschette nur an der in gefülltem Zustand quer zur Beatmungskanüle (1) verlaufenden gemeinsamen Auflagefläche miteinander verklebt sind.

9. Trachealtubus nach einem der vorhergehenden Ansprüche,
**dadurch gekennzeichnet,**
**daß** die Cuffmanschette (5) und die Tamponierblase (7) in Achsrichtung des Tubus um einen vorgegebenen Betrag verschiebbar sind.

10. Trachealtubus nach Anspruch 9,
**dadurch gekennzeichnet,**
**daß** die Cuffmanschette (5) und die Tamponierblase (7) durch eine Umschlagfalte (14) im Bereich der Befestigung an dem Tubus beweglich gelagert ist.

11. Trachealtubus nach einem der vorhergehenden Ansprüche,
**dadurch gekennzeichnet,**
**daß** das Fluid eine Flüssigkeit, vorzugsweise Wasser, ist, und daß die Einstellung des Druckes in der Tamponierblase (7) allein durch eine von dem Fluid unter Atmosphärendrcuck gebildete Flüssigkeitssäule erfolgt.

12. Trachealtubus nach einem der vorhergehenden Ansprüche,
**dadurch gekennzeichnet,**
**daß** der Innenraum der Tamponierblase (7) über einen Kanal mit großem Querschnitt mit einem Ausgleichsgefäß (11) verbunden ist.

13. Trachealtubus nach einem der vorhergehenden Ansprüche,
**dadurch gekennzeichnet,**
**daß** eine Druckquelle für die Cuffmanschette (5) unabhängig von der Fluiddruckquelle der Tamponierblase (7) betätigbar ist.

14. Trachealtubus nach einem der vorhergehenden Ansprüche,
**dadurch gekennzeichnet,**
**daß** ein die Tamponierblase (7) mit Fluid versorgender Kanal (8) im Bereich der Tamponierblase (7) mit mehreren Austrittsöffnungen (9) versehen ist, über die der Innenraum der Tamponierblase mit dem Kanal kommuniziert.

15. Trachealtubus nach einem der vorhergehenden Ansprüche,
**dadurch gekennzeichnet,**
**daß** die Tamponierblase (7) aus einem latexhautähnlichen, bei Faltenwurf leicht an sich selbst haftenden Material besteht.

16. Trachealtubus nach einem der vorhergehenden Ansprüche,
**dadurch gekennzeichnet,**
**daß** die Tamponierblase präformiert ist und in ungefülltem Zustand einen größeren Durchmesser als der Durchmesser des subglottischen Raumes aufweist.

## Claims

1. Tracheal tube sealing the trachea in liquid-tight fashion, with a fixing cuff (5) blocking the trachea beneath the glottis (3), through which a respiratory cannula (1) is led, wherein cranially, above the cuff (5), a tampon-bladder (7) of flexible material which can be enlarged by supply of fluid is arranged, which when filled is different in shape from the shape of the cuff (5),
**characterized in**
**that** in its filled state the tampon-bladder (7) immediately adjoins the cuff (5) and consists of foil-like material and is designed in its filled state to occupy the subglottal space completely.

2. Tracheal tube according to Claim 1,
**characterized in**
**that** the tampon-bladder (7) is designed in such a way that it completely occupies the supraglottal as well as the subglottal space.

3. Tracheal tube according to Claim 1 or 2,
**characterized in**
**that** the tampon-bladder (7) is preformed and adapted to the internal morphology of the larynx and the glottis.

4. Tracheal tube according to Claim 3,
**characterized in**
**that** the tampon-bladder (7) as viewed from the cuff (5) has a narrowing cross-section and, subsequent thereto, a cross section widening again preferably internally.

5. Tracheal tube according to any of the preceding Claims,
**characterized in**
**that** the tampon-bladder is located at a hose-like positioning element enabling the bladder to be positioned.

6. Tracheal tube according to any of the preceding Claims,
**characterized in**
**that** the tampon-bladder (7) encloses the cuff (5) with its wall.

7. Tracheal tube according to any of the preceding Claims,
**characterized in**
**that** the wall of the tampon-bladder (7) is attached, preferably by adhesive, to the wall of the cuff, in at least part of the area of their common opposing surface.

8. Tracheal tube according to Claim 7,
**characterized in**
**that** the tampon-bladder (7) and the cuff are stuck to each other only on the common opposing surface running at right angles to the ventilating cannula (1) in their filled condition.

9. Tracheal tube according to any of the preceding Claims,
**characterized in**
**that** the cuff (5) and the tampon-bladder (7) can, to a predetermined extent, be slid axially to the tube.

10. Tracheal tube according to Claim 9,
**characterized in**
**that** the cuff (5) and tampon-bladder (7) are movably placed through a surrounding fold (14) in the region of the attachment to the tube.

11. Tracheal tube according to any one of the foregoing Claims,
**characterized in**
**that** the fluid is a liquid, preferably water, and that the adjustment of the pressure in the tampon-bladder (7) is made solely by a liquid column formed by the fluid under atmospheric pressure.

12. Tracheal tube according to any of the foregoing Claims,
**characterized in**
**that** the inner cavity of the tampon-bladder (7) is connected via a channel of a large cross-section with an equalizing receptacle (11).

13. Tracheal tube according to any of the foregoing Claims,
**characterized in**
**that** a pressure source for the cuff (5) can be operated independently of the fluid pressure source of the tampon-bladder (7).

14. Tracheal tube according to any of the foregoing Claims,
**characterized in**
**that** a channel (8) supplying the tampon-bladder (7) with fluid is equipped in the region of the tampon-bladder (7) with a number of outlet apertures (9) via which the interior of the tampon-bladder communicates with the channel.

15. Tracheal tube according to any of the foregoing Claims,
**characterized in**
**that** the tampon-bladder (7) is made of a material resembling a latex skin, which is easily self-adherent when folded.

16. Tracheal tube according to any of the foregoing Claims,
**characterized in**
**that** the tampon-bladder is preformed and in its unfilled condition has a larger diameter than the diameter of the subglottal space.

## Revendications

1. Tube endotrachéal, qui ferme la trachée de manière étanche aux liquides, présentant un manchon de fixation (5) bloquant la trachée en-dessous de la glotte (3), par lequel est introduite une canule de respiration (1), un ballonnet tampon (7) en matériau flexible qui peut être agrandi par apport de fluide étant disposé en cranial au-dessus du manchon (5), lequel ballonnet par sa forme à l'état rempli est différent de la forme du manchon (5),
**caractérisé en ce que**
le ballonnet tampon (7) à l'état rempli est en appui direct sur le manchon et est constitué de matériau du type film et est formé, dans sa taille à l'état rempli, pour combler l'espace subglottique.

2. Tube endotrachéal selon la revendication 1,
**caractérisé en ce que**
le ballonnet tampon (7) est conformé de manière telle qu'il comble l'espace aussi bien subglottique que supraglottique.

3. Tube endotrachéal selon la revendication 1 ou 2,
**caractérisé en ce que**
le ballonnet tampon (7) est préformé et est adapté à la morphologie interne du larynx et de la glotte.

4. Tube endotrachéal selon la revendication 3,
**caractérisé en ce que**
le ballonnet tampon (7) à partir du manchon (5) présente une section transversale se rétrécissant et, se raccordant à celle-ci, une section transversale s'élargissant à nouveau de préférence intérieurement.

5. Tube endotrachéal selon l'une des revendications précédentes,
**caractérisé en ce que**
le ballonnet tampon est prévu sur un élément de placement du type tuyau permettant le placement du ballonnet.

6. Tube endotrachéal selon l'une des revendications précédentes,
**caractérisé en ce que**
le ballonnet tampon (7) par sa paroi entoure le manchon (5).

7. Tube endotrachéal selon l'une des revendications précédentes,
**caractérisé en ce que**
la paroi du ballonnet tampon (7) est assemblée, de préférence par collage, à la paroi du manchon, du moins dans une zone partielle de leur surface d'appui commune.

8. Tube endotrachéal selon la revendication 7,
**caractérisé en ce que**
le ballonnet tampon (7) et le manchon ne sont collés l'un à l'autre qu'à leur surface d'appui commune s'étendant transversalement à la canule de respiration (1) uniquement à l'état rempli.

9. Tube endotrachéal selon l'une des revendications précédentes,
**caractérisé en ce que**
le manchon (5) et le ballonnet tampon (7) sont capables de translation dans une mesure donnée, dans la direction axiale du tube.

10. Tube endotrachéal selon la revendication 9,
**caractérisé en ce que**
le manchon (5) et le ballonnet tampon (7) sont supportés mobile par un pli rabattu (14) dans la zone de la fixation sur le tube.

11. Tube endotrachéal selon l'une des revendications précédentes,
**caractérisé en ce que**
le fluide est un liquide, de préférence de l'eau, et **en ce que** le réglage de la pression dans le ballonnet tampon (7) s'opère uniquement par l'intermédiaire d'une colonne de liquide formée par le fluide à la pression atmosphérique.

12. Tube endotrachéal selon l'une des revendications précédentes,
**caractérisé en ce que**
l'espace intérieur du ballonnet tampon (7) est relié à un vase d'expansion (11) par un canal à grande section transversale.

13. Tube endotrachéal selon l'une des revendications précédentes,
**caractérisé en ce que**
une source de pression pour le manchon (5) peut être actionnée indépendamment de la source de pression de fluide du ballonnet tampon (7).

14. Tube endotrachéal selon l'une des revendications précédentes,
**caractérisé en ce que**
un canal (8) alimentant le ballonnet tampon (7) en fluide est pourvu dans la zone du ballonnet tampon (7) de plusieurs orifices de sortie (9), par l'intermédiaire desquels l'espace intérieur du ballonnet tampon communique avec le canal.

15. Tube endotrachéal selon l'une des revendications précédentes,
**caractérisé en ce que**
le ballonnet tampon (7) est constitué d'un matériau similaire à une peau en latex, adhérant facilement à lui-même en cas de formation de plis.

16. Tube endotrachéal selon l'une des revendications précédentes,
**caractérisé en ce que**
le ballonnet tampon est préformé et présente à l'état non rempli un diamètre supérieur au diamètre de l'espace subglottique.
